# EUROPÄISCHE PATENTSCHRIFT

(11) **EP 0 977 515 B1**
(45) Veröffentlichungstag und Bekanntmachung des Hinweises auf die Patenterteilung: **13.02.2008**
(21) Anmeldenummer: 98924187.2
(22) Anmeldetag: 23.04.1998
(51) Int. Cl.: A61B 17/72

(54) **KNOCHENDISTRAKTIONSVORRICHTUNG**
BONE TRACTION DEVICE
DISPOSITIF DE TRACTION POUR OS

(30) Priorität: 24.04.1997 DE 19717357
(43) Veröffentlichungstag der Anmeldung: 09.02.2000
(73) Patentinhaber: Betz, Augustin, 78479 Reichenau (DE)
(72) Erfinder: Betz, Augustin, 78479 Reichenau (DE)
(74) Vertreter: Thul, Stephan
(86) Internationale Anmeldenummer: PCT/EP1998/002413
(87) Internationale Veröffentlichungsnummer: WO 1998/047438

(56) Entgegenhaltungen:
- FR-A- 2 726 460
- US-A- 5 415 660
- US-A- 5 536 269
- US-A- 5 575 790
- US-A- 5 626 581
- J.GOTZ ET AL: "KONTINUIERLICHE VERLANGERUNG DES FEMUR BEI INTRAMEDULLARER STABILISIERUNG " ARCHIV FUER ORTHOPAEDISCHE UND UNFALL-CHIRURGIE, Bd. 82, Nr. 4, 1.Januar 1975, Seiten 305-310, XP000524031

## Beschreibung

Die vorliegende Erfindung betrifft eine Distraktionsvorrichtung zum Auseinanderbewegen zweier Teile eines Knochens, insbesondere zur Knochenverlängerung oder zur Überbrückung einer Knochenlücke gemäß dem Oberbegriff des Anspruchs 1, wie sie z.B. aus der FR-A-2726460 als bekannt hervorgeht.

Die Verwendung einer Distraktionsvorrichtung mit Marknagel ist aus der Druckschrift DE 39 21 972 C2 bekannt. Diese Vorrichtung dient insbesondere zur Verlängerung von Röhrenknochen oder Defektüberbrückung nach Trümmerbrüchen, einer Knochenentzündung oder nach Entfernen von Tumoren im Bereich langer Röhrenknochen.

Die Funktionsweise des bekannten Marknagels ist derart, daß durch Auseinanderbewegen der beiden Teile des Marknagels die beiden Teile des Knochens langsam auseinanderbewegt werden, wobei die sich dadurch bildende Lücke zwischen den beiden Knochenenden aufgrund des langsamen Vorschubes fortlaufend durch sich neu bildende Knochensubstanz überbrückt wird. Auf diese Weise können Knochen nicht nur verlängert werden, indem Knochen nach Aufbohren und Einführen des Marknagels in den Markraum durchtrennt und anschließend auseinandergezogen werden, sondern es können auch Knochenlücken überbrückt werden, indem ein von einem der Lücke benachbarten Knochenende abgetrenntes Knochenstück zum anderen Ende der Lücke bewegt wird. Entsprechend kann auch das Ende eines Knochenstumpfes, also ein Knochen mit fehlendem Knochenende, verlängert werden.

Distraktionsvorrichtungen sind auch aus der US-A-5 536 269, der zuvor genannten FR-A-2 726 460 und der US-A-5 415 660 bekannt, wobei bei diesen bekannten Vorrichtungen zwischen dem ersten Teil und dem zweiten Teil des Marknagels wirksame Sperrvorrichtungen vorhanden sind, die eine Rückbewegung der beiden gegeneinander verschobenen Teile verhindert.

Während gemäß US-A-5 536 269 als Antriebseinheit zur Erzeugung einer Relativbewegung zwischen den beiden Teilen des Marknagels eine KolbenZylindereinheit verwendet wird, ist es aus der FR-A-2 726 460 bekannt, die Relativbewegung zwischen den beiden zueinander beweglichen Teilen mittels eines stabförmigen Elements aus einer Formgedächtnislegierung zu realisieren.

Der Erfindung liegt die Aufgabe zugrunde, eine Distraktionsvorrichtung der eingangs genannten Art anzugeben, durch welche der Knochenwachstumsprozeß verbessert und eventuell auch beschleunigt werden kann. Außerdem soll die Belastung und Beeinträchtigung des Knochens verringert werden.

Diese Aufgabe wird gemäß der Erfindung durch das Zusammenwirken der Merkmale des Anspruchs 1 gelöst. Die abhängigen Ansprüche betreffen vorteilhafte Weiterbildungen der Erfindung.

Die Erfindung geht von der Erkenntnis aus, dass aufgrund des bei dem Auseinanderbewegen der Knochenteile mitbewegten Gewebes oder bei Belastung des entsprechenden Körperteils eine dieser Bewegung entgegengerichtete Kraft auftritt, die dazu führt, dass nach jedem Vorschubprozess das bewegte Knochenteil wieder zurückbewegt wird. Dies führt zu einer Verlangsamung und Verschlechterung des Knochenwachstumsprozesses. Durch die Erfindung wird eine Rückbewegung des bewegten Knochenteiles nach beendetem Vorschubprozess verhindert und der Knochenwachstumsprozess damit verbessert und beschleunigt.

Durch die nicht erfindungsgemäβe Ausgestaltung der beiden Teile des Marknagels als KolbenZylindereinheit, deren Teile durch einen Hydraulikantrieb gegeneinander verschiebbar sind, ist eine gegenüber bekannten Marknägeln verringerte Baugröße möglich. Der Durchmesser des Marknagels ist lediglich durch den Durchmesser des Kolbens und des Zylinders bestimmt, der bei Verwendung eines entsprechend großen Hydraulikdruckes so gering gewählt werden kann, daß die Distraktionsvorrichtung auch für kleinere Knochen, insbesondere Knochen der oberen Extremitäten eingesetzt werden kann. Der Hydraulikantrieb ermöglicht außerdem einerseits das Einbringen einer ausreichend großen Distraktionskraft und andererseits eine weitgehend unabhängige, also beliebige Anordnung der Antriebsvorrichtung für den Marknagel.

Nach der Erfindung ist einer der beiden Teile des Marknagels als äußeres Rohr ausgebildet.

Erfindungsgemäß umfaßt des automatische Antrieb ein Bauteil aus einer Formgedächtnislegierung . Durch vorgesehene Mittel zur Temperaturänderung kann der erste Teil des Marknagels gegenüber dem zweiten Teil verschoben werden. Die Erwärmung kann dabei über eine Heizspirale erfolgen, die wiederum von außerhalb des Körpers des Patienten, beispielsweise induktiv, mit Energie versorgt werden kann. Die Beeinträchtigung des Patienten wird damit wiederum sehr gering gehalten.

Nach der Erfindung greifen die beiden Teile des Marknagels ineinander und ist zwischen den beiden Teilen ein selbsthemmendes Gewinde vorgesehen, so dass bei Verdrehen des inneren Teils durch den automatischen Antrieb dieses gegen das äußere Teil axial verschoben, eine Rückbewegung jedoch verhindert wird. Als Antrieb dient ein Bauteil aus einer Formgedächtnislegierung, welches bei Temperaturänderung ein Drehmoment erzeugt. Das Bauteil kann beispielsweise als Spirale ausgebildet sein.

Für die Verwendung im Oberschenkelbereich weist der Marknagel der Distraktionsvorrichtung bevorzugt die Form eines runden Zylinders mit kontinuierlicher durchgehender Krümmung auf. Bei der Verwendung im Unterschenkelbereich ist es bevorzugt, wenn der Marknagel zwei abgewinkelte oder gekrümmte Enden aufweist. Diese Formen haben sich sowohl hinsichtlich der Belastung des Knochens als auch hinsichtlich der Funktion des Marknagels als besonders vorteilhaft erwiesen. Eine entsprechende Anpassung ist auch an die Knochen der oberen Extremitäten möglich und vorteilhaft.

Nach einer weiteren Ausgestaltung der Erfindung sind im Wesentlichen alle Teile der erfindungsgemäßen Distraktionsvorrichtung in das den Knochen enthaltende Körperteil implantierbar. Hierdurch wird die Gefahr von Infektionen durch Eindringen von Keimen von außen durch die Haut ausgeschlossen. Auch für das Befinden des Patienten ist die vollständige Unterbringung der Vorrichtung im betroffenen Körperteil von Vorteil. Die Antriebsenergie des Hydraulikantriebs kann entweder durch einen ebenfalls implantierbaren Energiespeicher oder von außen, beispielsweise induktiv, zur Verfügung gestellt werden.

Nach einer weiteren Ausgestaltung der Erfindung ist eine in den Körper des Patienten implantierbare Messeinrichtung für die Bewegung des Marknagels vorgesehen, insbesondere zur Messung von Druck, Dehnung, Drehmoment und Temperatur. Durch diese Messeinrichtung kann der Knochenaufbauprozess überwacht und protokolliert werden, ohne dass der Patient durchleuchtet werden muss und somit ohne die dadurch hervorgerufene Belastung des Patienten.

Zur Protokollierung der gemessenen Werte ist bevorzugt ein in den Körper implantierbarer Speicher vorgesehen. Des Weiteren kann ein ebenfalls bevorzugt in den Körper implantierbarer Sender vorgesehen sein, um die Messwerte nach außen zu übertragen. Hierdurch ist es möglich, ohne Eingriff in den Körper des Patienten die festgestellten Messwerte abzurufen und zu überprüfen. Aus diesen Messwerten können für die Behandlung wichtige Daten entnommen oder abgeleitet werden, wie beispielsweise die Distraktionsstrecke und der Distraktionsverlauf.

Nach einer weiteren Ausgestaltung der Erfindung ist eine ebenfalls bevorzugt in den Körper implantierbare Regeleinrichtung zur automatischen Regelung der Marknagelbewegung vorgesehen. Mit einer derartigen Regeleinrichtung kann die Marknagelbewegung optimiert und eine zu große Belastung des Patienten vermieden werden.

Weitere vorteilhafte Ausgestaltungen der Erfindung sind in den Unteransprüchen angegeben.

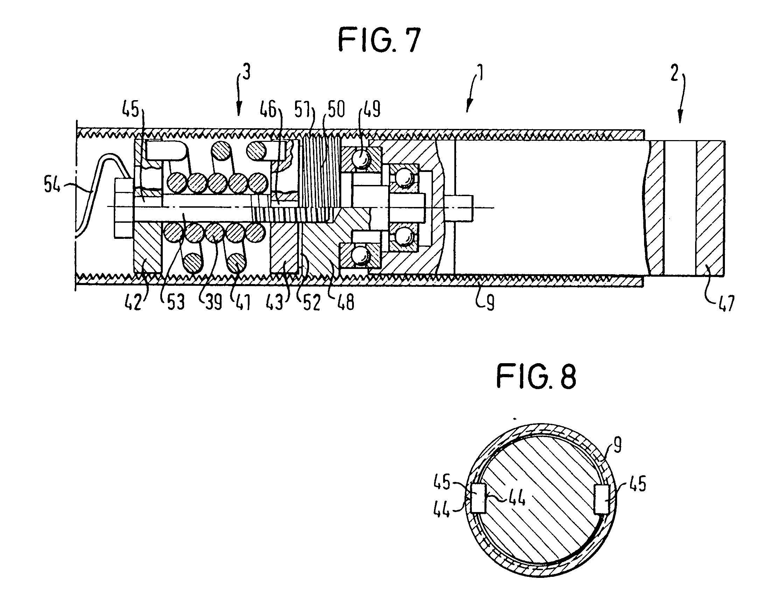

Ein Ausführungsbeispiel der Erfindung ist in der Zeichnung in den Figuren 7 und 8 dargestellt und wird nachfolgend beschrieben. Es zeigen, jeweils in schematischer Darstellung,
- Fig. 1: den grundsätzlichen Aufbau einer nicht-erfindungsgemäßen Distraktionsvorrichtung mit einem teilweise ausgefahrenen Marknagel in teilweise geschnittener Darstellung,
- Fig. 2: eine Fig. 1 entsprechende Darstellung einer Variante einer nicht-erfindungsgemäßen Distraktionsvorrichtung,
- Fig. 3: eine teilweise geschnittene Darstellung eines Teils des Marknagels einer weiteren Variante der nicht-erfindungsgemäßen Distraktionsvorrichtung,
- Fig. 4: eine Fig. 3 entsprechende Darstellung einer weiteren Variante der nicht-erfindungsgemäßen Distraktionsvorrichtung,
- Fig. 5: einen Querschnitt durch ein Detail einer nicht-erfindungsgemäßen Distraktionsvorrichtung,
- Fig. 6: eine teilweise geschnittene Darstellung des Marknagels einer nicht-erfindungsgemäßen Distraktionsvorrichtung,
- Fig. 7: einen Schnitt durch einen Teil eines Marknagels einer erfindungsgemäßen Distraktionsvorrichtung und
- Fig. 8: einen Querschnitt gemäß Linie AA in Fig. 7.

Eine nicht-erfindungsgemäße Distraktionsvorrichtung umfasst einen in den Markraum eines Knochens einführbaren Marknagel 1, welcher zwei axial auseinanderfahrbare, jeweils an einem der beiden Knochenteile befestigbare Teile 2, 3 aufweist, sowie eine hydraulische Antriebseinrichtung 4 zum Auseinanderfahren der beiden Teile 2, 3 des Marknagels 1. Der Hydraulikantrieb 4 ist im Körper des Patienten implantierbar und induktiv mit Antriebsenergie von außen versorgbar. Er umfasst einen Druckgeber 5, welcher über eine Hydraulikleitung 6 mit den beiden als KolbenZylindereinheit 8, 9 ausgebildeten Teilen 2, 3 des Marknagels 1 verbunden ist.

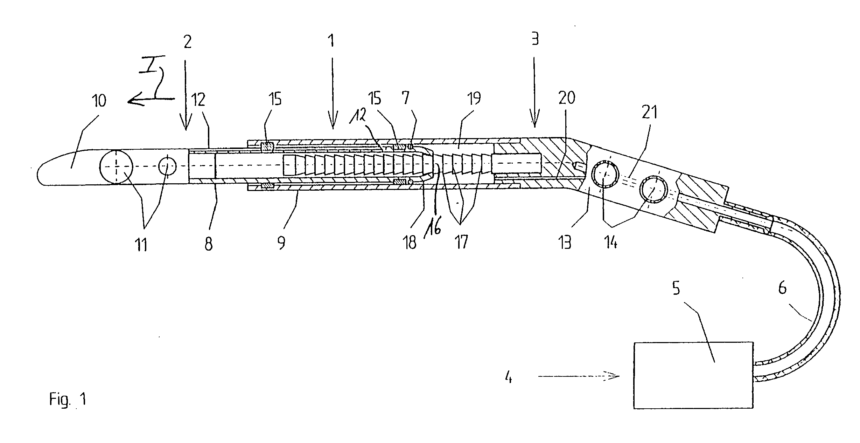

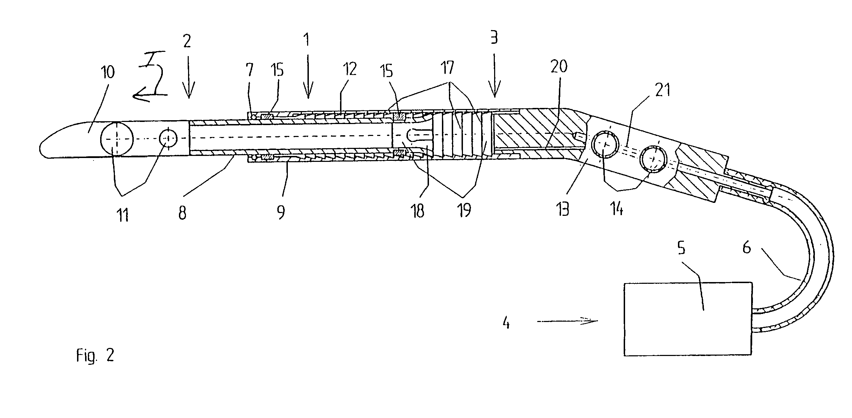

Bei den in den Fig. 1 und 2 dargestellten Varianten weist der nicht-erfindungsgemäße Marknagel zwei teleskopartig auseinanderfahrbare, über eine Ringdichtung 7 gegeneinander abgedichtete koaxiale Rohre 8, 9 auf, wobei das innere Rohr 8 an dem distalen Teil 2 und das äußere Rohr 9 an dem proximalen Teil 3 des Marknagels 1 angeordnet ist. Das innere Rohr 8 ist in ein anatomisch gekrümmtes distales Endstück 10 des Marknagels 1 eingesetzt, welches mit zwei Durchgangsbohrungen 11 versehen ist, in welche hier nicht dargestellte Befestigungsmittel, insbesondere Befestigungsschrauben einsetzbar sind, die an einem Knochenteil anbringbar sind. Eine der beiden Durchgangsbohrungen weist dabei einen dem Durchmesser des Endstücks annähernd entsprechenden Durchmesser auf. Das äußere Rohr 9 ist an einem ebenfalls anatomisch gekrümmten proximalen Endstück 13 angeordnet, welches zwei Durchgangsbohrungen 14 für ebenfalls an einem Knochenteil festlegbare Befestigungsmittel, insbesondere Schrauben, aufweist.

Bei der in Fig. 1 dargestellten Variante ist konzentrisch zu und innerhalb des äußeren Rohres 9 an dem proximalen Endstück 13 des Marknagels 1 eine Stange 16 angeordnet, auf welcher das innere Rohr 8 axial verschiebbar und radial dichtend geführt ist. Die Stange 16 ist auf ihrer Außenseite mit Rastausnehmungen 17 versehen, in welche an der im äußeren Rohr 9 liegenden Stirnseite des inneren Rohrs 8 vorhandene, elastisch gegen die Stange 16 vorgespannte Klauen 18 einrastbar sind. Wie dargestellt, sind mindestens zwei solche Klauen 18 auf voneinander abgewandten Seiten der Stange 16 vorgesehen. Entsprechend sind auf voneinander abgewandten Seiten der Stange 16 Paare von Ausnehmungen 17 vorgesehen, wobei mehrere Paare solcher Ausnehmungen 17 auf der Stange 16 axial hintereinander angeordnet sind, so dass die Klauen 18 beim Ausfahren des inneren Rohres 8 aus dem äußeren Rohr 9 nacheinander in mehrere solcher Paare von Ausnehmungen 17 einrasten können.

Durch die Ringdichtung 7, das innere Rohr 8, das äußere Rohr 9, das distale Endstück 10 und das proximale Endstück 13 wird ein Druckraum 19 gebildet. Der Druckraum 19 ist über einen Verbindungskanal 20 im proximalen Endstück 13 mit einer zentralen Bohrung 21 im proximalen Endstück 13 des Marknagels 1 verbunden. Über ein in Fig. 1 nicht dargestelltes Rückschlagventil ist die zentrale Bohrung 21 an die Hydraulikleitung 6 angeschlossen.

Bei der in Fig. 2 dargestellten Variante sind die Rastausnehmungen 17 auf der Innenumfangsseite des äußeren Rohres 9 vorgesehen. Entsprechend weisen die am proximalen Ende des inneren Rohres 8 vorhanden Klauen 18 federnd nach außen. Eine zentrale Stange ist bei dieser Ausgestaltung daher nicht erforderlich.

Bei beiden Varianten der Fig. 1 und 2 ist das innere Rohr 8 zudem mit einer Passfedernut 12 versehen, in welche ein am äußeren Rohr 9 vorhandener Gleitring 15 mit einem entsprechenden Vorsprung eingreift, um das innere Rohr 8 gegenüber dem äußeren Rohr 9 gegen ein Verdrehen zu sichern.

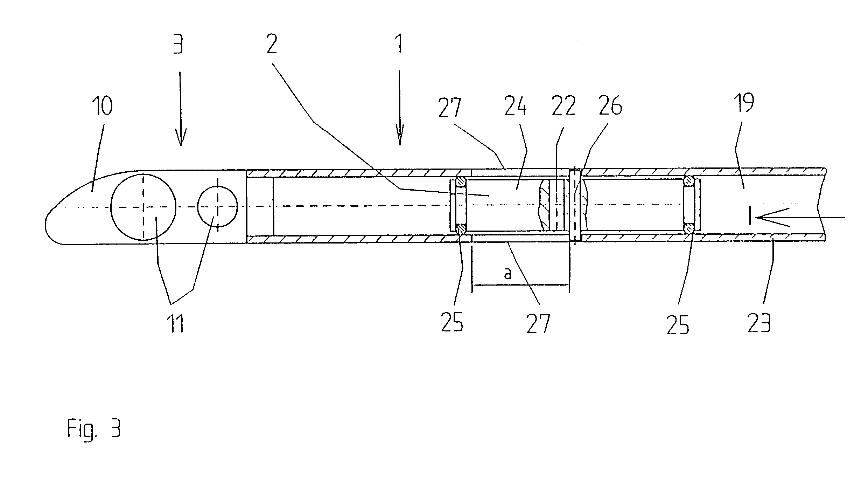

Bei der in Fig. 3 dargestellten Variante eines nicht-erfindungsgemäßen Marknagels 1 ist in einem einen bevorzugt kreisförmigen Querschnitt aufweisenden Zylinder 23 ein Kolben 24 verschiebbar geführt. Der Kolben 24 ist mittels zweier an seinen beiden Enden vorgesehener Ringdichtungen 25 gegenüber dem Zylinder 23 abgedichtet. Auf der proximalen Seite des Kolbens 24 ist im Zylinder 23 ein Druckraum 19 ausgebildet, welcher über einen nicht dargestellten Hydraulikanschluss mit einem Hydraulikantrieb verbindbar ist. Durch Zufuhr von Druckfluid in den Druckraum 19 ist der Kolben 24 auf der proximalen Seite mit einem Hydraulikdruck beaufschlagbar und dadurch im Zylinder 23 zum distalen Ende hin verschiebbar.

Etwa in der Mitte ist der Kolben 24 mit einem beidseits radial über seinen Außenumfang vorstehenden Führungsstift 26 versehen, welcher in zwei in Axialrichtung des Marknagels 1 verlaufende Langlöcher 27 in den jeweils gegenüberliegenden Wänden des Zylinders 23 eingreift. Über den Führungsstift 26 ist der Kolben 24 im Zylinder 23 gegen ein Verdrehen gesichert. Zugleich wirkt der Führungsstift 26 als Anschlag für die Vorschubbewegung des Kolbens 24 im Zylinder 23.

An dem distalen Ende des Zylinders 23 ist wiederum ein Endstück 10 vorgesehen, welches mit zwei Durchgangsöffnungen 11 versehen ist, in welche hier nicht dargestellte Befestigungsmittel zur Befestigung des Zylinders 23 an einem Knochen einsetzbar sind. Entsprechend ist der Kolben 24 mit einer zum Führungsstift 26 parallelen Durchgangsöffnung 22 versehen, die mit den Langlöchern 27 fluchtet. So kann ein an einem Knochenstück verankertes Befestigungsmittel durch die Durchgangsöffnung 22 hindurchgeführt und das Knochenstück an dem Kolben befestigt werden.

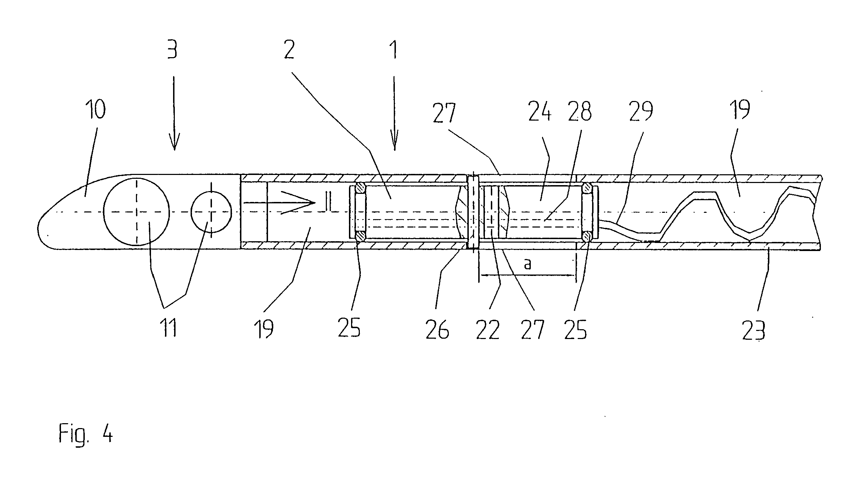

Die in Fig. 4 dargestellte weitere Variante eines nicht-erfindungsgemäßen Marknagels 1 stimmt weitgehend mit der Variante von Fig. 3 überein. Der wesentliche Unterschied besteht darin, dass hier der Kolben 24 zusätzlich mit einem achsparallelen Hydraulikkanal 28 versehen ist, an welchen eine Hydraulikdruckleitung 29 anschließbar ist. Auf diese Weise kann der auf der distalen Seite des Kolbens 24 zwischen diesem und dem Zylinder 23 gebildete Raum 19 mit Hydraulikdruck beaufschlagt werden. Der Kolben 24 wird bei dieser Variante also, wie mit Pfeil II angegeben, vom distalen Ende zum proximalen Ende des Marknagels 1 hin verschoben, während er bei der zuvor beschriebenen Variante gemäß Pfeil I vom proximalen Ende zum distalen Ende hin verschoben wird. Entsprechend ist bei der Variante von Fig. 4 die Durchgangsöffnung 22 auf der proximalen Seite des Führungsstiftes 26 vorgesehen, während sie sich bei der Variante von Fig. 3 auf der distalen Seite befindet.

Die Funktionsweise der erfindungsgemäßen Vorrichtung ist in allen Fällen derart, dass der Marknagel 1 in den Markraum eines Röhrenknochens eingeführt wird, wobei bevorzugt ein Aufbohren des Markraums nicht erforderlich ist. Nach dem Einführen des Marknagels 1 werden dessen Teile 2 und 3 mit zwei auseinanderzubewegenden Teilen des Knochens verbunden. Gegebenenfalls wird der Knochen zur Bildung zweier solcher Teile an geeigneter Stelle durchtrennt.

Bei der in Fig. 1 und 2 dargestellten Distraktionsvorrichtung wird nach Einbringen und Festlegen des Marknagels 1 der Hydraulikantrieb 4 betätigt, um die Teile 2, 3 des Marknagels 1 auseinanderzubewegen. Aus dem Druckgeber 5 wird Druckfluid über die Hydraulikleitung 6 zum Marknagel 1 gefördert, wo es über das Rückschlagventil, die zentrale Bohrung 21 und den Verbindungskanal 20 im proximalen Endstück 13 zum Druckraum 19 gelangt. Der erzeugte Hydraulikdruck wirkt auf die Stirnseite des inneren Rohres 8 und drückt dieses teleskopartig aus dem äußeren Rohr 9 heraus, wobei das innere Rohr 8 mit seinen Klauen 18 in die Rastausnehmungen 17 der Stange 16 bzw. des äußeren Rohres 9 einrastet, sobald diese miteinander fluchten. Die Rastausnehmungen 17 sind dabei so verteilt, dass nach jedem geplanten Vorschubvorgang bis zum maximalen Verschiebeweg der beiden Teile 2, 3 des Marknagels 1 gegeneinander ein Einrasten möglich ist, um eine Rückbewegung des inneren Rohres mit dem distalen Endstück 10 in das äußere Rohr 9 zu verhindern. Durch den Abstand der Raststellen ist jedoch eine Rückbewegung um eine geringe gewünschte Strecke möglich.

Über das mit dem inneren Rohr 8 ausgeschobene distale Endstück 10 wird der daran befestigte Knochenteil von dem an dem proximalen Endstück 13 befestigten Knochenteil entfernt. Dies geschieht derart langsam und in zeitlichen Intervallen, dass sich in der entstehenden Lücke Knochensubstanz bilden kann, welche die Lücke mit der Zeit verschließt. Nach Beendigung der Distraktion ist dadurch der Knochen um ein entsprechendes Stück verlängert worden.

Die Verwendung der in den Fig. 3 und 4 dargestellten Varianten erfolgt grundsätzlich in derselben Weise wie bei der zuvor beschriebenen Variante, wobei der Marknagel von Fig. 4 eine Bewegung eines Knochenteils vom distalen zum proximalen Ende ermöglicht.

Das in Fig. 5 dargestellte Rückschlagventil ist zur Anordnung in einem proximalen Endstück 13 eines Marknagels 1 vorgesehen. In einem Halter 35 ist eine Druckkugel 30 gelagert, die durch eine im Halter 35 abgestützte Feder gegen einen Dichtring 31 gepresst wird. Der Dichtring 31 ist in einer Druckplatte 32 gelagert, welche in einer Ausnehmung 38 des proximalen Endstücks 13 verschiebbar und mittels eines Dichtrings 37 dichtend geführt ist. Der über die Druckkugel 30 auf den Dichtring 31 ausgeübten Kraft wirkt dabei die Kraft einer Federanordnung 33 entgegen, die zwischen Druckplatte 32 und proximalem Endstück 13 angeordnet ist.

Über zentrale Ausnehmungen der Federanordnung 33 und der Druckplatte 32 ist die Dichtkugel 30 mit über die Hydraulikleitung 6 herangeführtem Druckfluid beaufschlagbar. Die Hydraulikleitung 6 ist dabei über einen Druckkegel 36 unter radialer Komprimierung im distalen Endstück 13 verankert. Bei entsprechendem Hydraulikdruck hebt die Dichtkugel 30 vom Dichtring 31 unter Komprimierung der Feder 34 ab, so dass Druckfluid zwischen Dichtkugel 30 und Dichtring 31 vorbeiströmen und das Rückschlagventil passieren kann. Sobald der Druck abfällt, schließt das Rückschlagventil wieder, indem die Dichtkugel 30 von der Feder 34 gegen den Dichtring 31 gepresst wird.

Bei einer Krafteinwirkung auf den Marknagel 1 entgegen der Distraktionsrichtung wird das Druckfluid im Druckraum 19 unter Druck gesetzt. Durch das Rückschlagventil wird dabei ein Rückfließen von Druckfluid aus dem Druckraum 19 in die Hydraulikleitung 6 verhindert. Gleichzeitig ermöglichen die Federn 33 eine gewisse Verschiebung der Druckplatte 32 und damit des distalen Teiles 2 des Marknagels 1. Auf diese Weise kann trotz blockierter Rückbewegung des Marknagels 1 eine gewünschte kleine Rückbewegung stattfinden, um das Knochenwachstum anzuregen.

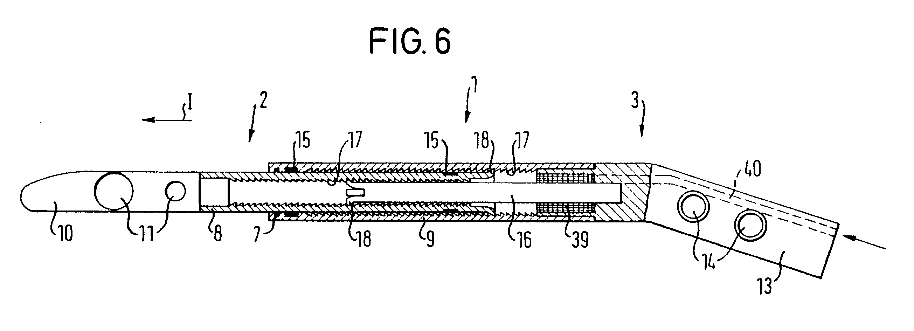

Bei der in Figur 6 dargestellten Variante der nicht-erfindungsgemäßen Distraktionsvorrichtung ist sowohl das äußere Rohr 9 als auch das innere Rohr 8 auf der Innenseite mit Rastausnehmungen 17 versehen. Die Stange 16, auf welcher das innere Rohr 8 geführt ist, und das innere Rohr 8 weisen jeweils an ihrem innen liegenden Ende Klauen 18 auf, die in die zugeordneten Rastausnehmungen eingreifen.

Im Bereich des im äußeren Rohr 9 festgelegten Endes der aus einer Formgedächtnislegierung gebildeten Stange 16 ist eine Heizspirale 39 vorgesehen, über welche die Stange 16 erwärmbar ist. Die Energieversorgung der Heizspirale erfolgt über entsprechende, nicht dargestellte Leitungen, die über eine Ausnehmung 40 im proximalen Endstück 3 des Marknagels 1 zur Heizspirale 39 geführt sind. Die Energieversorgung selbst erfolgt bevorzugt induktiv von außerhalb des Körpers des Patienten.

Bei Energiezufuhr zu der Heizspirale 39 erwärmt diese die Stange 16, die sich daraufhin aufgrund der Materialeigenschaften der Formgedächtnislegierung erheblich ausdehnt. Eine Ausdehnung kann beispielsweise etwa 7 % der Gesamtlänge der Stange 16 betragen. Über die Klauen 18 und die Rastausnehmungen 17 des inneren Rohres 8 wird dieses beim Ausdehnen der Stange 16 mitgenommen und aus dem äußeren Rohr 9 ausgefahren. Nach Abschalten der Energiezufuhr zur Heizspirale 19 kühlt die Stange 16 wieder ab, insbesondere auf die Körpertemperatur des Patienten. Die Stange 16 zieht sich wieder zusammen, wobei die Klauen 18 über die Rastausnehmungen 17 des inneren Rohres 8 ratschen. Eine Rückbewegung des inneren Rohres 8 wird dabei durch die Klauen 18 des inneren Rohres 8 verhindert, die in die Rastausnehmungen 17 des äußeren Rohres 9 eingreifen. Durch zyklisches Erwärmen der Stange 16 kann so das innere Rohr 8 aus dem äußeren Rohr 9 ausgeschoben werden. Das heißt, das distale Endstück 2 des Marknagels 1 wird aus dem proximalen Endstück 3 teleskopisch ausgefahren und dadurch die beiden Knochenteile auseinander bewegt.

In entsprechender Weise kann auch eine umgekehrte Bewegung stattfinden. Insbesondere kann ein derartiger Antrieb mit vom Gedächtnislegierung auch bei einem Marknagel gemäß den Figuren 3 und 4 eingesetzt werden.

Die in Figur 7 und 8 dargestellte Variante einer erfindungsgemäßen Distraktionsvorrichtung verwendet ebenfalls ein Bauteil aus einer Formgedächtnislegierung. In diesem Ausführungsbeispiel handelt es sich jedoch um eine Spirale 41 aus einer solchen Legierung, die sich bei Erwärmung in Spiralrichtung verlängert und dadurch ein Drehmoment erzeugt.

Die Spirale 41 ist im äußeren Rohr 9 angeordnet und mit ihrem einen Ende an einem in dem äußeren Rohr 9 axial verschiebbar, aber drehfest geführten ersten Element 42 und mit ihrem anderen Ende an einem zweiten Element 43 festgelegt. Das zweite Element 43 ist ebenfalls im äußeren Rohr 9 verschiebbar geführt. Im Gegensatz zum ersten Element 42 ist das zweite Element 43 aber um einen kleinen Winkel gegenüber dem äußeren Rohr 9 verdrehbar geführt. Hierfür weisen die beiden Elemente 42 und 43 sowie das äußere Rohr übereinstimmende Passfedernuten 44 auf, in die einerseits eine breite Passfeder 45 zwischen erstem Element 42 und äußerem Rohr 9 und andererseits eine schmale Passfeder 46 zwischen zweitem Element 43 und äußerem Rohr 9 eingreifen. Zwischen schmaler Passfeder 46 und Passfedernut 44 verbleibt somit ein Freiraum, der ein Verdrehen des zweiten Elementes 43 gegenüber dem äußeren Rohr 9 um einen kleinen Winkel zulässt.

Auf der in der Spirale 41 abgewandten Seite des zweiten Elementes 43 ist in dem äußeren Rohr 9 eine ausfahrbare Stange 47 vorhanden, die in dem äußeren Rohr 9 drehfest und gleitend geführt ist. Hierfür ist auch zwischen der Stange 47 und dem äußeren Rohr 9 eine in entsprechende Passfedernuten 44 eingreifende breite Passfeder 45 vorgesehen. Zwischen der Ausschubstange 47, die insbesondere dem distalen Ende des Marknagels 1 zugeordnet, und dem zweiten Element 43 ist ein drittes Element 48 vorgesehen, welches drehbar mit der Ausschubstange 47 verbunden ist. Hierfür ist zwischen dem dritten Element 48 und der Ausschubstange 47 ein Axiallager 49 vorhanden. Das dritte Element 48 ist an seinem Umfang mit einem Außengewinde 50 versehen, welches in ein auf der Innenseite des Außenrohres 9 vorhandenes Innengewinde 51 eingreift.

Zwischen dem dritten Element 48 und dem zweiten Element 43 ist eine Stirnverrastung vorgesehen, die nur in einer Drehrichtung des zweiten Elementes 43 gegen das dritte Element 48 wirkt. Schließlich ist das erste Element 42 über eine Axialschraube 53, welche die beiden Elemente 42 und 43 durchgreift und mit dem dritten Element 48 verbunden ist. Innerhalb der Spirale 41 ist eine Heizspirale 39 angeordnet, die über eine Leitung 54 mit Energie beaufschlagbar ist.

Über die Heizspirale 39 wird die Spirale 41 aus vom Gedächtslegierung erwärmt, die sich daraufhin in Spiralrichtung verlängert und das zweite Element 43 entsprechend verdreht. Das zweite Element 43 nimmt über die Stirnverrastung 52 das dritte Element 48 mit, welches seinerseits die Stange 47 aufgrund des Eingriffs seines Außengewindes 50 in das Innengewinde 51 des äußeren Rohres 9 definiert verschiebt. Über die Axialschraube 53 wird auch das erste Element 42 mitgenommen. Nach Wiederabkühlen der Spirale 41 verkürzt sich diese und dreht das zweite Element 43 in seine Ausgangslage zurück. Wegen der einseitig ausgebildeten Stirnverrastung 52 wird dabei das dritte 48 und die Ausschubstange 47 nicht mitgenommen. Diese verbleiben vielmehr in ihrer ausgeschobenen Lage. Einer Rückbewegung der Ausschubstange 47 bei äußerer Belastung wirkt die selbsthemmende Eigenschaft der beiden miteinander in Eingriff stehenden Gewinde 50 und 51 entgegen. Ein Wiederzusammenfahren des Marknagels ist dadurch also gesperrt.

Nicht dargestellt ist die ebenfalls in das den Knochen enthaltende Körperteil implantierbare Sensorik, durch welche der Behandlungsverlauf kontrolliert werden kann, ohne dass der Patient durchleuchtet werden muss, was dessen Belastung weiter verringert. Insbesondere wird eine Kraft- und Wegmessung durchgeführt, beispielsweise mittels eines Dehnungsmessstreifens oder eines potentiometrisch arbeitenden Längenmesssystems. Die Sensorik kann auch dazu verwendet werden, ein Regelsystem für den Vorschub des Marknagels auszubilden, insbesondere für die Vorschubgeschwindigkeit. Der erfindungsgemäße Marknagel kann mit Querschnitten von 10 mm und kleiner ausgebildet werden und eignet sich daher besonders auch für kleinere Knochen, also Knochen der oberen Extremitäten oder kindlichen Knochen.

Ebenfalls nicht dargestellt ist die Möglichkeit eines Telemetriesystems, um während des Einsatzes des Marknagels über entsprechende Messeinrichtungen festgestellte Daten wie Druck, Dehnung, Drehmoment und Temperatur aus dem Körper des Patienten nach außen zu übertragen. Zusätzlich kann ein Speicher vorgesehen sein, der ebenso wie der Sender bevorzugt im Körper des Patienten implantierbar ist. In diesem Speicher werden die Daten für einen Abruf von außen gespeichert. Die Aktivierung des Telemetriersystemes kann durch einen Reed-Schalter mit Hilfe eines Magneten erfolgen, der innerhalb der Sendespule untergebracht ist. Gegebenenfalls können noch Sicherungsmechanismen eingebaut werden, um eine Fehlaktivierung durch äußere Störungen zu vermeiden.

## Patentansprüche

1. Distraktionsvorrichtung zum Auseinanderbewegen zweier Teile eines Knochens, insbesondere zur Knochenverlängerung oder zur Überbrückung einer Knochenlücke,
mit einem in den Markraum eines Knochens einführbaren Marknagel (1), welcher zwei axial gegeneinander verschiebbare, jeweils an einem der beiden Knochenteile befestigbare Teile (2, 3) aufweist, einer automatischen Antriebseinrichtung (4) mit einem Bauteil (41) aus einer Formgedächtnislegierung zum Verschieben der beiden Teile (2, 3) des Marknagels (1) gegeneinander,
einer zwischen dem ersten Teil (2) und zweiten Teil (3) des Marknagels (1) wirksamen Sperrvorrichtung (17, 30, 31, 50, 51), die eine Rückbewegung der beiden gegeneinander verschobenen Teile (2, 3) verhindert , wobei
eines der beiden ineinandergreifenden Teile (2, 3) des Marknagels (1) als äußeres Rohr ausgebildet ist, **dadurch gekennzeichnet, dass** zwischen den beiden Teilen (2, 3) ein selbsthemmendes Gewinde (51, 51) vorgesehen ist, so dass bei Verdrehen des inneren Teils (2) durch den automatischen Antrieb dieses gegen das äußere Teil (3) axial verschoben, eine Rückbewegung jedoch verhindert wird, und
dass der automatische Antrieb ein Bauteil (41) aus einer Formgedächtnislegierung umfasst, welches bei Temperaturänderung ein Drehmoment erzeugt.

2. Distraktionsvorrichtung nach Anspruch 1,
**dadurch gekennzeichnet,**
**dass** das als Antrieb wirkende Bauteil als Spirale (41) ausgebildet ist, die mit ihrem einen Ende an einem in dem äußeren Teil (3) axial verschiebbar, aber drehfest geführtem ersten Element (42) und mit ihrem anderen Ende an einem zweiten Element (43) festgelegt ist, welches ebenfalls im äußeren Teil (3) verschiebbar, aber um mindestens einen kleinen Winkel verdrehbar geführt ist und mit dem inneren Teil (2) des Marknagels (1) eine in nur einer Richtung wirkende Drehverbindung, insbesondere Stirnverrastung (52) aufweist, und
**dass** das erste Element (42) mit dem inneren Teil (2) des Marknagels (1) drehbar verbunden ist.

3. Distraktionsvorrichtung nach einem der vorhergehenden Ansprüche,
**dadurch gekennzeichnet,**
**dass** der Marknagel (1) ein distales Endstück (10) mit einer abgerundeten Spitze und einer Durchtrittsöffnung (11) für einen Bolzen aufweist, der an einem Knochenteil befestigbar ist, wobei der Querschnitt der Durchtrittsöffnung (11) annähernd so groß wie der Durchmesser des Endstückes an dieser Stelle ist und dass vorzugsweise eine zweite Durchtrittsöffnung (11) mit einem demgegenüber verhältnismäßig kleinen Querschnitt im Endstück (10) vorgesehen ist.

## Claims

1. A traction apparatus for the moving apart of two parts of a bone, in particular for bone extension or for bridging a bone gap,
including a medullary nail (1) which can be introduced into the medullary space of a bone and which has two parts (2, 3) which are mutually axially displaceable and can each be fastened to one of the two bone parts;
an automatic drive device (4) having a component (41) made from a shape memory alloy for the mutual displacement of the two parts (2, 3) of the medullary nail (1);
a blocking device (17, 30, 31, 50, 51) which is effective between the first part (2) and the second part (3) of the medullary nail (1) and which prevents a return movement of the two mutually displaced parts (2, 3),
wherein one of the interengaging parts (2, 3) of the medullary nail (1) is made as an outer tube,
**characterised in that**
a self-locking thread (51, 51) is provided between the two parts (2, 3) so that, on the rotation of the inner part (2), it is axially displaced relative to the outer part (3) by the automatic drive, but a return movement is prevented; and
**in that** the automatic drive includes a component (41) made of a shape memory alloy which generates a torque on a change in temperature.

2. A traction device in accordance with claim 1, **characterised in that** the component acting as the drive is made as a spiral (41) whose one end is fixed to a first element (42) guided axially displaceably, but rotationally fixedly, in the outer part (3) and whose other end is fixed to a second element (43), which is likewise displaceable in the outer part (3), but is guided rotatably over at least a small angle and has a rotary connection, in particular an end face latch (52), acting in only one direction with the inner part (2) of the medullary nail (1); and **in that** the first element (42) is rotatably connected to the inner part (2) of the medullary nail (1).

3. A traction device in accordance with any one of the preceding claims, **characterised in that** the medullary nail (1) has a distal end piece (10) with a rounded tip and a passage opening (11) for a pin which can be fastened to a bone part, with the cross-section of the passage opening (11) being approximately as large as the diameter of the end piece at this position; and **in that** a second passage opening (11) is preferably provided in the end piece (10) with a cross-section which is comparatively small with respect thereto.

## Revendications

1. Dispositif écarteur pour déplacer deux parties d'un os en éloignement l'une de l'autre, en particulier pour l'allongement d'un os ou pour combler une lacune dans un os,
comprenant un clou médullaire (1) susceptible d'être introduit dans le canal médullaire d'un os et comportant deux parties (2, 3) susceptibles d'être déplacées axialement l'une par rapport à l'autre et d'être fixées chacune sur l'une des deux parties de l'os, et
comprenant un dispositif d'entraînement automatique (4) avec un composant (41) en un alliage à mémoire de forme pour déplacer les deux parties (2, 3) du clou médullaire (1) l'une par rapport à l'autre,
comprenant un dispositif de blocage (17, 30, 31, 50, 51) agissant entre la première partie (2) et la seconde partie (3) du clou médullaire (1) et empêchant un mouvement de retour des deux parties (2, 3) déplacées l'une par rapport à l'autre,
dans lequel l'une des deux parties (2, 3), engagées l'une dans l'autre, du clou médullaire (1) est réalisée sous forme de tube extérieur,
**caractérisé en ce qu'**il est prévu entre les deux parties (2, 3) un pas de vis (51, 51) à autofreinage, de sorte que par rotation de la partie intérieure (2) par l'entraînement automatique, celle-ci est déplacée axialement par rapport à la partie extérieure (3), mais qu'un mouvement de retour est empêché, et **en ce que** l'entraînement automatique comprend un composant (41) en alliage à mémoire de forme, qui engendre un couple rotatif lors d'une modification de température.

2. Dispositif d'écartement selon la revendication 1,
**caractérisé en ce que** le composant faisant office d'entraînement est réalisé sous forme de spirale (41) immobilisée par une extrémité sur un premier élément (42) capable de translation axiale dans la partie extérieure (3), mais guidé solidairement en rotation, et par son autre extrémité sur un second élément (43) qui est également guidé en translation dans la partie extérieure (3), mais capable de tourner au moins d'un petit angle, et dans lequel la partie intérieure (2) du clou médullaire (1) présente une liaison rotative agissant dans une seule direction, en particulier un enclenchement frontal (52), et **en ce que** le premier élément (42) est relié en rotation à la partie intérieure (2) du clou médullaire (1).

3. Dispositif d'écartement selon l'une des revendications précédentes,
**caractérisé en ce que** le clou médullaire (1) comprend un embout distal (10) avec une pointe arrondie et une ouverture traversante (11) pour un boulon susceptible d'être fixé sur une partie de l'os, la section transversale de l'ouverture traversante (11) est approximativement aussi grande que le diamètre de l'embout à cet emplacement, et **en ce qu'**il est de préférence prévu une seconde ouverture traversante (11) avec une section comparativement petite par rapport à la précédente dans l'embout (10).
